# EUROPEAN PATENT SPECIFICATION

(11) **EP 0 910 388 B1**
(45) Date of publication and mention of the grant of the patent: **18.09.2002**
(21) Application number: 97916696.4
(22) Date of filing: 27.03.1997
(51) Int. Cl.: A61K 31/715, A61K 31/29, A61K 33/24

(54) **PHARMACEUTICAL PREPARATION FOR THE TREATMENT OF GASTRITIS, REFLUX OESOPHAGITIS, DUODENITIS, DYSPEPSIA AND ULCER DISEASE**
ARZNEIPRÄPARAT ZUR BEHANDLUNG DER GASTRITIS, DER REFLUX-ÖSOPHAGITIS, DUODENITIS,DYSPEPSIE UND ULKUSERKRANKUNGEN
PREPARATIONS PHARMACEUTIQUES POUR LE TRAITEMENT DE LA GASTRITE, DE L'OESOPHAGITE DE REFLUX, DE LA DUODENITE, DE LA DYSPEPSIE ET DE L'ULCERE

(30) Priority: 29.03.1996 SE 9601219
(43) Date of publication of application: 28.04.1999
(73) Proprietor: Krotkiewski, Marcin, S-436 00 Askim (SE)
(72) Inventor: Krotkiewski, Marcin, S-436 00 Askim (SE)
(74) Representative: Hammond, Andrew David
(86) International application number: SE9700544
(87) International publication number: WO97036599

(56) References cited:
- EP-A- 0 348 143
- WO-A-95/25521
- GB-A- 2 272 375
- US-A- 4 199 560

## Description

### BACKGROUND OF THE INVENTION

Duodenal and gastric ulcer disease, GERD (gastric oesophageal reflux disease) and common gastritis are chronic conditions with exaceberations and remissions occurring over periods of 30 years or more. The H2-receptor antagonists were introduced in about 1976 and are apart from known side effects well tolerated and reasonably effective in terms of ulcer healing.

More recently, acid pump inhibitors, such as Omeprazol (Losec^{R}) have been shown to inhibit gastric acid secretion to a greater extent to work more quickly and to heal a higher percentage of duodenal/gastric ulcers. The principal problem is that once duodenal/gastric ulcer is healed and GERD ameliorated most duodenal/gastric ulcers and GERD with heartburns will recur. About 80% of peptic ulcers can be expected to recur within one year and almost 100% within two years after stopping the course of treatment.

The explanation of the high recurrence rate of GERD is most probably associated wuth the colonisation of oesophageal and gastric mucose by pathogenic Campylobacter (Heliobacter pylori). Already in the eighties several studies showed that the likelihood of duodenal/gastric ulcer's recurrence was dramatically reduced if the associated Heliobacter pylori infection had been eradicated.

Bell and Power (Scand. J. Gastroent. 1993; 196: 7-11) summarizing the data from eight studies showed that when Heliobacter pylori is eradicated (i.e. when organism is still undetected 6-24 months after treatment) duodenal ulcer relapse is 3% as compared to 65% in duodenal ulcer patients in whom the organism is still present.

Virtually all duodenal ulcer patients are Heliobacter pylori (HP) positive. About 70% of gastric ulcers show the presence of HP. However recent studies show that the eradication of HP is a condition sine qua non for a reduction of the recurrance rate of duodenal/gastric ulcers.

Graham et al (Am. Intern. Med. 1992; 116: 705-708) randomised 26 patients with HP positive gastric ulcer to receive either Ranitidine alone or triple therapy (tetracycline, metrondiazole and bismuth subsalicylate) plus Ranitidine. After one year of follow-up the ulcer recurrence rate was 74% in patients treated with Ranitidine alone, compared with only 13% in those treated with triple therapy plus Ranitidine.

In a recent German multicenter gastric ulcer study, 130 patients were randomised to receive either Omeprazol 20 mg daily or triple therapy consisting of bismuth subsalicylate 600 mg three times daily for 8 weeks, plus Amoxicillin 500 mg twice daily and Tinidazol 1 g daily for days 1-10 (BayerdÜrffer et al, J. Med. Sci. 1992; 161: Suppl. 10:30). The ulcer relapse rate at 6 months was only 3% in patients rendered HP negative after therapy compared with 33% in those who remained HP positive.

Thus bismuth salts have been found to cause the effective eradication of of HP together with the healing effect on oesophageal and gastric mucose and the long-term improvement of the clinical symptom of GERD.

The most known bismuth preparation De NolR consists of bismuth subcitrate bound to a peptide and is creating a colloidal solution coating the oesophageal and gastric mucose in a way allowing the effective eradication of HP and the healing process of the mucose. However other casual factors are most probably involved in the pathogenesis of GERD.

One of the most important factor is the decrease of the contracting ability of the lower oesophagus sphincter LES - causing reflux regurgitation of the highly acid content of the stomach to the lower part of oesophagus. The oesophagus mucose exposed for detoriating effect of the gastric juice become irritated and finally ulcerous due to the persistent and repeated reflux (experienced subjectively as heartburns).

To ameliorate the heartburns, the reflux and the regurgitations the alginic preparation Gaviscone^{R} has been used since many years. GavisconeR consists of alginic acid in combination with sodium bicarbonate and creates in the stomach a special foam, "umbrella-like" hampering the content of the stomach to enter the oesophagus. In the same time the GavisconeR is buffering the acid gastric juice and remains present in the stomach during a relatively long time.

GB-A-2 272 375 refers to a pharmaceutical preparation for the treatment of gastrointestinal disturbances. The preparation contains a mixture of alginic acid and a metal compound, e g bismuth nitrate.

FR-M-1728 also refers to pharmaceutical preparation containing a mixture of alginic acid and bismuth nitrate.

WO 95/25521, refers to a composition for treating upper gastrointestinal tract distress containing bismuth subsalicylate as active ingredient. Alginic acid is mentioned as one example of a disintegrating agent to be used in the composition. It is stated in the publication that the composition is no prolonged dosis composition, instead it is designed for quick dissolution in the stomach and absorption into the blood stream.

### SUMMARY OF THE INVENTION

The object of the present invention is to provide a new preparation for the treatment of gastritis, reflux oesophagitis, duodenitis, dyspepsia and ulcer disease. It should be effective in causing eradication of Campylobacter such as Heliobacter pylori (HP) and further it should actively prevent the reflux of the stomach content.

The same preparation is even aimed for the treatment and prevention of other infections diseases of digestive tract -as travellers diarrhoea, and other types bacterial enterocolitis.

This object has according to the invention been achieved by a pharmaceutical preparation which contains a mixture of a bismuth citrate and an alginate and any reaction products obtained, especially bismuth alginate. The combination of alginate and bismuth will enable both mucous protection and eradication of Campylobacter such as HP.

The preparation according the the invention may further contain one or more antibiotic substances in order to give higher and more stable eradication rate. Such antibiotic substances may be Amoxicillin^{R} and/or Metronizadol.

Preferably the preparation further contains an agent, e g sodium bicarbonate, that in the acid milieu in the stomach will cause foaming of the bismuth alginate at the same time as it provides a buffering effect on the acid gastric juice.

### DESCRIPTION OF THE INVENTION

The bismuth alginate preparation according to the invention is less toxic to known bismuth preprations due to its slow absorption rate and lower increases of the bismuth plasma concentrations. The bismuth ions are firmly bound to the alginate which will prevent or at least reduce possible bismuth nephrotoxic effects, or other systemic effects as reported in the medical literature for other easily absorbable bismuth preparations. The bismuth is acting locally, but is passing substantially unabsorbed through the digestive tract.

The bismuth alginate preparation according to the present invention can be prepared by mixing ammonium bismuth citrate (K,NH₄)₅ Bi₆(OH)₁₁(C₆H₅O₇) or ammonium bismuth subcitrate with sodium-, potassium- and/or calcium alginate. Sodium bicarbonate (NaHCO₉) may be added for obtaining neutralization of the gastric acid milieu and for its foaming effect. Calcium chloride may be concurrently added to the reaction medium for creating beads of calcium alginate. The purpose of this microcapsulation procedure is to slow the dissociation and diffusion process of the bismuth alginate. Coating with calcium alginate is aimed to decrease the quick diffusion and dissociation of the bismuth from the bismuth alginate and its further absorption to the blood stream. High plasma concentrations of bismuth are prerequisite for the occurence of the complications described for different bismuth preparations.

The bismuth alginate can be used alone in monotherapy of GERD due to its high H. pylori eradication rate. It can also be used combined with antimicrobial agents in double, triple and quadriple therapy. Such antimicrobial agents are Amoxicillin^{R} and/or Metronizadol (Tinidazol^{R}). These antimicrobial agents may either be combined with the bismuth alginate in one preparation or given separately. Bismuth alginate can also be used in combination with sucralfate in double and in combination with sucralfate and amoxicillin in triple therapy.

According to our Study 5 reported below which was a one year follow-up study with blood serology after 1, 6 and 12 months only one patient in a group treated with Biogastrin (a bismuth alginate preparation according to the invention) + Amoxicillin^{R} and none of the Metronizadol sensitive patients treated with Biogastrin + Tinidazol^{R} (Fasigyn Pfizer) showed a positive blood serology while more than 30% of the patients treated with a combination of Losec^{R} with Amoxicillin^{R} showed indices of reinfection (positive serology). It is worth to note that H. pyroli does not develop resistance to bismuth as it does to Metronizadol and Amoxicillin^{R}.

The study shows that Biogastrin + Amoxicillin^{R} and Biogastrin + Tinidazol^{R} are superior to Omeprazol + Amoxicillin^{R} in terms of eradication of H. pyroli and comparable in terms of amelioration of GERDs symptomatology.

No antimicrobial therapy (Amoxicillin^{R}, Fluoroquinolanes, Metronidazol etc) provides a satisfactory eradication rate when given alone. Metronidazol given as a monotherapy leads almost always to drug resistance. Resistance can be substantially decreased when Metronidazol (Tinidazol^{R}) is combined with bismuth alginate (Biogastrin).

Experience has also shown that although it is relatively easy to clear the organism temporarily it is much more difficult to eradicate it, perhaps because the organism lives in the gastric mucous and gastric pits where antibiotic levels may not reach bactericidal levels. This means that once administration of the antibiotic stops the few remaining bacteria that have been "lurking" in sanctuaries sites reproduce, and the number of bacteria reaches a level that can be detected.

In the case of GERD even monotherapy with bismuth alginate can be recommended with the expected eradication rate of HP by 40-60%, but with the substantial remission of heartburns and dyspeptic symptoms. Bismuth alginate as monotherapy is superior to both Gaviscone^{R} and De Nol^{R} (CBS- Collodial Bismuth Substrate) when given alone (Study 5 below).

### ALCOHOL - HELIOBACTER PYLORI

Helicobacter pylori species possess verty active alcohol dehydrogenase (ADH). Even in the presence of very low ethanol concentration as after ingesion of so called low-alcoholic beverages the very toxic (free radicals producing carcinogenic and immunogenic) acetaldehyde (ACH) is produced in HP infected patients. ACH binds readily to gastric mucosal proteins. Formation of ACH protein adducts leads to cellular damage and induce an immune response with gastric mucosal injury as a consequence. Furthermore, ACH inhibit proliferation of gastric epithelial cells in antral mucous. This most probably is to treat as an important pathogenic factor inhibiting normal gastric mucosal protection and repair. There is a growing evidence regarding the association of alcohol with the presence of Helicobacter pylori. Different bismuth compounds even at the very low concentration specifically inhibit the active of cytosolic Helicobacter pylori ADH by 93 %!

Preliminary evidence seems to indicate that the addition of Omeprazol to Biogastram / Biogastrozol increases substantially the eradication rate in NIR (Nitroimidozole - Metronizadol) resistant patients.

Thus, Biogastrin is to be treated as a drug of choice for all patients with GERD or NUG (Nonulcer Gastritis) and peptic or ulcer disease who consume some amount of alcohol, especially for those who feel increase of heartburn's or the detoriation of other dyspeptic symptoms after drinking even a small amount of wine or other alcoholic beverages.

### UREA - AMMONIA

Helicobacter pylori is characterised by high levels of urease activity, which metabolises urea to ammonia. It has been proposed that ammonia is cytotoxic to gastric mucosal cells (Bawer, M.R. et al J. Clin. Pathol. 1988;41:597) because hydrogen back diffusion is increased (Hazell, S.L. Lee.A.Lancet. 1986:15-17) and because acid secretion is stimulated by serum concentration of gastric (Switch, J.T.L. et al. Gut. 1990;31: 522-525). The gastric mucosa of Helicobacter pylori infected persons in characterised by a large accumulation of neutrophils (Morns, A et al. Am. J.Gastroenterolog. 1987; 82; 192-199). Activated neutrophils generate the superoxide anion and hydrogen peroxides.

Myeloperoxidase, which is concomitantly secreted into the extracellular space, can catalyse the oxidation of chloride by hydrogen peroxide to yield hypochlorous acid and monochloramine. Monochloramine is reported to be exceptionally reactice and toxic because of its highly hipophilic properties and low molecular weight /Thomas, E.L. Grisham, M.D, Jeffersson, M.M. Method Enzymolog. 1986; 132: 585-593). Mucarami et al. Gastroenterology 1990, 98: A210 and Morishita et al. Europ. J. Gastroenterol. & Hepat. 1993; 5. Suppl. 1: 5133-5136) recently showed that antineutrophil serum and taurine (which binds monochloramine) protect the rats and human from gastric mucosal damage induced by ammonia and Heliocobacter pylori (in patients with gastric ulcers).

Bismuth alginates os buffering ammonia and due to its scavening activity has a clear protective effect on gastric mucose apart of its bactericidal activity.

### ADMINISTRATION

The bismuth alginate and the antimicrobial agents can be administered separately or in the same preparation. The administration form is either tablets or capsules coated with a slow-release agent, e g calcium alginate or liquid form. All substances added to the preparation should be pharmaceutically acceptable and non-toxic in the added amounts.

When given as monotherapy a non-binding example of an appropriate dosage of bismuth alginate would be 1-2 capsules 3 times a day each containing 500 mg of bismuth alginate.

A dosage example of bismuth alginate in combination with Amoxillin would be 2 capsules 3 times daily each capsule containing 500 mg bismuth alginate and 250 mg Amoxicillin.

A dosage example of bismuth alginate in combination with Fimidazol^{R} and Amoxicillin would be 2 capsules 3 times daily each capsule containing 250 mg bismuth alginate + 250 mg Timidazol^{R} + 250 mg Amoxicillin.

As mentioned above the antimicrobial agents may alternatively be administered separately. Dosage for bismuth alginate in combination with sucralfate is bismuth alginate 500 mg together with 1 g sucralfate three times daily. In the triple therapy together with amoxicillin 250 mg three times daily.

### EXAMPLE

In all the studies below there was used a preparation named Biogastrin which was prepared by mixing the following substances:

| | | |
|---|---|---|
| Protanal LF 200 RB | 40.00 g | |
| Ammonium bismuth citrate 10301 | 345.00 g | |
| Providon K30 | | 95.00 g |
| Calcium chloride | 20.00 g | |
| | | 500.00 g |

Protanal LF 200 RB was obtained from Pronova, Drammen, Norway. Instead of Protanal LF 200 RB any other alginate of different viscosity originating from Sea-weeds (algae) of type laminaria and/or ascophylum nodosum produced of any manufacturing unit processing Sea-weeds can be used in similar way.

Ammonium bismuth citrate 10301 containing 43-461 bismuth was obtained from Riedel-de Haën. Any other producer of ammonium bismuth citrate or ammonium bismuth subcitrate can be used. The final product can be coated with lecithin and if needed further microcopsultated to create granulate.

Granulate can be used in sachets. Alternatively the calcium alginate beads containing bismuth alginate can be put in capsules directly.

In vitro study of susceptibility of H. pylori to bismuth alginate (Biogastrin) and bismuth subcitrate (De NolR)

Biogastrin was first dissolved and diluted in physiologic saline into concentrations of 0, 1, 2, 4, 8, 16, and 32 mg/l. These solutions were mixed with brucella agar and of them, seven culture plates were casted. In them, samples of ten different strains of HP were dropped (Table 1). The same was done with DE-NOL (Table 2). The plates were incubated for 48 hours in 35 centigrades using an athmosphere consisting of oxygen (5%), carbon dioxid (10%) and nitrogen (85%). Thereafter, the growth of HP was determined visually. The lowest concentration in which no HP growth was observed, was regarded as the MIC.

For the inoculum, ten strains of HP were inoculated into beef stock, incubated for 24 hours and thereafter diluted into the density 0,5 on the MacFarland-scale.

The MICs were determined twice; in January and February 1994.
The Biogastrin, bismuth-alginate, was supplied by Krotex International AB, Askim, Sweden and DE-NOL by Algol, Helsinki, Finland.

### RESULTS

The MIC of Biogastrin ranged from 4 mg/l to 32 mg/l, the median being 16 mg/l in the study performed in January 1994 and 8 mg/l on the study performed in february 1994. The MIC of DE-NOL ranged from 4 mg/l to 8 mg/l, the median being 8 mg/l in both studies. The results are presented in detail at Tables 1 and 2. Biogastrin contains 34% of metallic bismuth and DE-NOL 90%.

**TABLE 1**

| The suspectibility of HP to Biogastrin in studies performed in January and February 1994. | | | | | | |
|---|---|---|---|---|---|---|
| HP strain | Concentration of Biogastrin | | | | | |
| | 1 mg/l | 2 mg/l | 4 mg/l | 8 mg/l | 16 mg/l | 32 mg/l |
| N C T C 11637 | 3/3 | 3/3 | 3/3 | 3/3 | 1/1 | 0/0 |
| N C T C 11638 | 3/3 | 3/3 | 3/2 | 3/0 | 0/0 | 0/0 |
| 1009/89 | 3/3 | 3/3 | 0/0 | 0/0 | 0/0 | 0/0 |
| 138/90 | 3/3 | 3/3 | 3/3 | 3/3 | 1/0 | 0/0 |
| 970/93 | 3/3 | 3/3 | 3/3 | 3/3 | 1/0 | 0/0 |
| 939/93 | 3/3 | 3/3 | 3/3 | 0/0 | 0/0 | 0/0 |
| 1139/93 | 3/3 | 3/3 | 3/0 | 3/0 | 0/0 | 0/0 |
| 1204/93 | 3/3 | 3/3 | 3/3 | 3/0 | 0/0 | 0/0 |
| 1209/93 | 3/3 | 3/3 | 3/3 | 3/2 | 0/0 | 0/0 |
| 1045/90 | 3/3 | 3/3 | 3/3 | 0/0 | 0/0 | 0/0 |
| N C T C 11637 | 3/3 | 3/3 | 3/3 | 3/3 | 0/0 | 0/0 |
| N C T C 11638 | 3/3 | 3/3 | 3/2 | 3/0 | 0/0 | 0/0 |

Intensity of growth: 0, 1, 2 and 3 in increasing scale. The first figure denotes growth in test performed in January 1994, the latter in February 1994.

**TABLE 2**

| The suspectitibility of HP to DE-NOL in studies performed in January and February 1994. | | | | | | |
|---|---|---|---|---|---|---|
| HP strain | Concentration of DE-NOL | | | | | |
| | 1 mg/l | 2 mg/l | 4 mg/l | 8 mg/l | 16 mg/l | 32 mg/l |
| N C T C 11637 | 3/3 | 3/3 | 3/3 | 1/1 | 0/0 | 0/0 |
| N C T C 11638 | 3/3 | 3/3 | 3/2 | 0/0 | 0/0 | 0/0 |
| 1009/89 | 3/3 | 3/3 | 0/0 | 0/0 | 0/0 | 0/0 |
| 138/90 | 3/3 | 3/3 | 2/3 | 0/0 | 0/0 | 0/0 |
| 970/93 | 3/3 | 3/3 | 2/3 | 1/0 | 0/0 | 0/0 |
| 939/93 | 3/3 | 3/3 | 3/1 | 0/0 | 0/0 | 0/0 |
| 1139/93 | 3/3 | 3/3 | 3/1 | 0/0 | 0/0 | 0/0 |
| 1204/93 | 3/3 | 3/3 | 3/3 | 0/0 | 0/0 | 0/0 |
| 1209/93 | 3/3 | 3/3 | 3/3 | 0/0 | 0/0 | 0/0 |
| 1045/90 | 3/3 | 3/3 | 0/0 | 0/0 | 0/0 | 0/0 |
| N C T C 11637 | 3/3 | 3/3 | 3/2 | 0/0 | 0/0 | 0/0 |
| N C T C 11638 | 3/3 | 3/3 | 3/2 | 3/0 | 0/0 | 0/0 |

Intensity of growth: 0, 1, 2 and 3 in increasing scale. The first figure denotes growth in test performed in January 1994, the latter in February 1995.

As the weigth percentage of bismuth in Biogastrin is 34 %, and in DE-NOL 90%, the MIC medians must be corrected in order to enable relevant comparison. After correction, the medians of Biogastrin are 5.44 and 2.72 mg bismuth/l and that of DE-NOL 7.20 mg/l. Thus the suspectability of HP to Biogastrin is very comparable to that of DE-NOL. Although Biogastrin in this set-up even showed superior MICs to that od DE-NOL, this difference is very unlikely significant. The visual determinationof MICs has bias towards optimistic MICs and also hampers the relevant comparisonof these drugs.

Nevertheless, Biogastrin seems to have good antihelicobacter efficiency in vitro.

### STUDY No. 1

The aim of the study was to compare the therapeutic effects of Biogastrin with that of Cimetidine (Tagamet) in a group of patients with the longlasting and relatively therapy resistent reflux.

### Patients and methods:

Twenty patients - 8 men and 12 women were allocated to the study. The inclusion criteria were age; under - 65, the lack of any cardiovascular, kidney, or metabolic disease (diabetes), not other medication than that for the treatment of gastrosophageal reflux and the at least five years long history of refluxesophagitis with the therapy resistent symptoms of heartburns and regurgitation. The diagnostic verfication of the advanced reflux oesophagitis has been done by means of:
a. endoscopic examination
b. intraesophageal ph monitoring during 24 hours (known from the previous stays in the hospital)
c. detailed register of all symptoms reported by patients, (See description above page 3, 5).

All patients were asked to score their clinical symptoms heartburn, regurgitation, postural syndrome by means of visual analogue scale-VAS for each symptom.
To verify the presence of campylobacter the biopsy samples have been taken during endoscopy.

The samples were than assessed:
a histologically
b. by culturing for gram staining and urease activity

All patients when allocated to the study have been treated with Tagamet with our without antiacids and this therapy was given to them during the last six months preceeding the study. All patients have been subjected to the endoscopic examination and found to have the endoscopic picture corresponding to grades 2 to 3 (according to modified grading of Savary and Miller - for definition see description above page 4). None of the patients were receiving antibiotics at the time of examination.

### DESIGN OF THE STUDY.

After giving informal verbal consent the patients were randomly allocated to a double blind protocol either to the group receiving cimetidine (Tagamet) 800 g at night and Biogastrin (2 capsules 3 times daily - 30 minutes after each big meal) or to the group on cimitidene and placebo.

(This was the first study and we did not dare to withdraw the Tagamet. The intention of this study was thus to evaluate wether the addition of Biogastrin to the already implemented treatment with H2 receptor blockers in the therapy resistent patients would improve the therapeutic effect in comparison with placebo).

The therapy as above was discontinued after 3 weeks, the patients who showed improvement after this period were then followed additionally three weeks while the cimentidine treatment was discontinued and only Biogastrin treatment maintained (in the same dosage 3 capsules 3 times daily). The non improved patients maintained their previous Tagamet treatment.

In the placebo group the non improved patients received during this period (period B) the same therapy.

### RESULTS

Ot the 10 patients treated with cimetinine and Biogastrin eight showed a clear improvement in the endoscopic examination. While at the start of the study three had the grade 3, four the grade 2 and three the grade 1 (according to Savary and Miller), eight patients showed normal or almost a normal picuter of oesophageal mucose after three weeks of treatment with Tagamet and Biogastrin. On the contary in the placebo + Tagamet group six patients shows no improvement, one improved from grade 2 to 1 and three grade 3 to 2.
The difference was thus significant and evident. The mean number of oesophagel erosions or ulcers decreased from 4.8 + 0.5 to 3.8 + 0.4 in the Placebo - Tagamet group.

Positive oesophageal culture for CP (campylobacter pyloris) were found in nine (and positive histology on eight) cases.

Six of the nine patients were allocated to the group with the combined treatment Biogastrin + Tagemet. Whenever there was a positive capylobacter culture the treatment with Biogastrin + Tagemet resulted in the eradication of CP with the negative culture urease test and improved histology.
In contrast to this group three initially CP positive patients remined positive after the termination of the three weeks treatment - placebo + Tagamet.

Period B was followed in the open way (not double blind).
Of the six unimproved patients (previously at the placebo + Tagamet), all showed significant symptomatic and endoscopic improvement. (Threee improved from grade to 0, one from grade 3 to 1 and two from grade 2 to 0). Three patients with the positive culture for CP after period A became negative after institution of Biogastrin treated during period B. All patients on the Biogastrin alone during the period B (totally 12 patients) showed no symptomatic signs of the recurrens and generally no symptoms of the reflux oesophagitis.

It is concluded that the implementation of Biogastrin in the patients with the chronic, therapy resistent reflux oesophagitis receiving countinously H₂ receptor blockers in associated with the clear symptomatic improvement of the condition (decrease of the heartburns and regurgitations).
The subjective symptomatologic improvement (as verified by VAS ratings) was associated with improvement in the endoscopic picture of oesophageal mucose and the eradication of the campylobacter pylori.
In patients who showed improvement after the first three weeks of treatment (Tagamet + Biogastrin) the continuation of treatment with the Biogastrin alone appeared to be sufficient to maintain the previously obtained symptom free condition.

It is suggested that the addition of Biogastrin to the treatment arsenal of previously therapyresistent patients is to be treated as the promising new therapeutic approach. In the cases the culturing for CP is positive, the presence of CP in biopsy material constitutes a clear indication for the institition of Biogastrin treatment.

### STUDY No. 2.

The aim of this study was planed as comparison of the therapeutic effect of sucralfate and that of Biogastrin. Both preparation share several similarities in terms of healing effect on mucose, binding properties to the gastric and eosophagel epithelium, effect on prostaglandins synthesig, and protective effect fot the acid content of the reflux. To provoke the exacerbation of the symptoms, the previously adequatly treated patients received unbuffred aspiring during two week. In this specific study we used the temporally provoked exacerbation of the symptoms to make the groups more comparable and easier to match.

Twenty-two patients were recruited for the clinical assessment of the effects of the sucralfate (Succosa Hässle Mölndal, Sweden) or Biogastrin. All patients were looking after the medical care for the control of their chronic dyspepsia and esophagitis (Gastroesophageal reflux disease (GERD) with the main symptoms of heartburns and regurgitation requiring sporadic medication with antiacids. To provoke the exacerbation of the symptoms all 22 patients received unbuffred aspirin 500 mg two times daily during two weeks.

In the same time patients received either sucralfate 1 g - 4 times daily as 10 ml dos sachets containing 10 ml mixture (group § 11 patients) or Biogastrin 1 g three times daily (in form of 3 ml colloidal solution), group B (11 patients). After the first weeks the group S changed sucralfate to Biogastrin and group B - Biogastrin to Sucralfate. The study was conducted in the double blind manner.

Before the start of the study all patients underwent the endoscopic examination - verifying the grade 1-2 non erosive, non ulcerated, non stenotic oesophagitis or GERD (which was treated as the inclusion criterium). Anothers criterium was the absence of any medication apart of antiacids and the lack of any cardiac, kidney, liver or metabolic disease. The age of patients varied between 28-62 years (mean 37+8) and body weight between 61-87 kg (mean 74+6) and BMI 22-28 kg/m (means 24.2+3.1 1 kg/m.

Before the cross-over of the groups, all patients awaited one week without any medication.

### RESULTS

After the first week of Aspirin administration seven patients in the group S and 1 patient in the group B reported epigastric burning and frequent regurgitation.
During the second week all patients in the group S who reported heartburn and regurgitations during the first week, reported remarkable improvement of their symptoms after the installation of Biogastrin treatment during the period B.
This study revealed that the Biogastrin is superior to sucralfate in protection against the influence of typical prostaglandins synthesis blockers provoking typical symptoms of reflux esophagitis. Biogastrin could be used there preferably in form of colloidal solution.

### STUDY No. 3

This study was thought as the complementary pilot trial to the study No. 1.
The aim of this study was to confirm the previous finding that the therapy resistent patients with the reflux oesophagitis can be treated with Biogastrin.
Seven patients suffering from heartburns and frequent regurgitation in spite of long-term treatment with Ranitidine (Zantac Glaxo) - 150 mg 2 times daily, have been examined with the routin endoscopic. In three patients endoscopy revealed the grade 3 and in the four grade 2 of the endoscopic picture (according to Miller and Savary).

All seven patients received Biogastrin 1 g four times daily (and Zantac as previously) during one week. Out of seven patients - five reported clear relief of their symptoms - these five patients received their Biogastrin also during additional furhter six weeks as a single treatment. All patients were able to maintain the ameliortion of their symptoms unchanged. The endoscopic reexamination has been performed after the third and after the six and twelve weeks of treatment.

After the first six weeks three patients the endoscopic examination revealed improvement from grade 3 to grade 1 and from grade 2 to 0 in the other two. After additional six weeks of treatment no pathological change could be detected at endoscopy in the esophageal and gastric mucose.

This study - similary to study No. 1 showed that Biogastrin can be useful in the patients resistent to the habitual treatment with H₂ receptor blockers. The addition of Biogastrin to the routine treatment (Zantac or Tagamet) was associated with the rapid improvement which allowed further single therapy with Biogastrin alone.

### STUDY No. 4

The aim of the study was to assess the therapeutic effect of Biogastrin versus Gaviscon with respect to the main indication of Gaviscon - reflux - regurgitation and epigastric heartburns pain/discomfort. As previously, because of the initially small experience with Biogastrin we did not dare to design the study in classical manner i.e. double blind crossover, the medical team responsible for the treatment choose thus the stepwise, more safe form of clinical assessment.

Sixteen patients with symptomatologic (actual and by medical history) and endoscopic verified reflux esophagitis treated continously with Ranitidine (Zantac - Glaxo 150 mg 2 times daily) and alginic acis (Gaviscone - Ferring) 2 g 4 times daily have been allocated to the study.
All patients reported some reflux problems and/or heart burns at least 3 times a day in spite of treatment. The inclusion of criterium was reccurent treatment and periods of acute oesophagitis during the last 4 years. All patients were examined endoscopically and have been shown to have the edoscopic picture grade 3 to 2 (according to Savary and Miller).

All patients gave their informed consent and changed then the Gaviscone to the Biogastrin 1 g (3 ml) three times daily. Of the sixteen patients who attended the study twelve reported the market improvement with respect to their heartburns and 10 reported being totally free from regurgitations.
Endoscopy performed four weeks after the change of the treatment revealed that in 10 patients endoscopic picture improved from grade 3 to grade 2 and in 6 patients from grade 1 to 0 (no pahtological changes).
After additional 4 weeks of treatment all but one patient showed the endoscopic grade of 0. Twelve patients in this group could stop the treatment with Ranitidin after the first four weeks and maintain the treatment with Biogastrin alone during subsequent 4 weeks. Additional two patients did the same because of the distinct amelioration of their symptoms after the first six weeks of treatment staying on the Biogastrin only during the last two weeks.

This study confirmed the efficacy of Biogastrin with respect to the amelioration of the typical symptomatology of reflux oesophagitis. The result seem to indicate that Biogastrin acts most probably similar to Gaviscon in terms of creating a plug of the colloidal Bismuth alginate strengthening the natural protection of the body (lower oesophageal sphincter) around hiatus, preventing the exposure if the sensitive oesophageal mucose for the acid content of the gastric juice (moving up during reflux regurgitaion).

In summary, it seems probably that the significant improvement obtaining in several therapy resistent patients - (obtaining otherwise adequate treatment) is dependent on the specific effect of Biogastrin on campylobacter. (The selection of patients for the studies and their therapy resistance can indicate that the CP infected patients were overrepresented in the material of patients who attended the studies). The colloidal "plug" of Bismuth alginate influencing the reflux regurgitations is most probably contributing to the amelioration of the symptoms.
Further studies are needed for the confirmation of the preliminary results.

### STUDY No. 5

Omeprazol and Omeprazol + Amoxicillin has been recently shown to be affective in amelioration of symptoms and eradication of Helicobacter pylori in GERD (gastroeosophageal reflux disease). However, this form of therapy requiers continuos treatment due to the recurrance rate both with respect to subjective symptoms (reflux with heartburn's) and to the reoccurrence (recolonisation) of Helicobacter pylori.

Biogastrin has been shown to diminish the insensitivity to Metronidazol and to increase the eradication rate both with double, triple and quadruple therapy. The aim of the present study was to study the long-term effects of two weeks therapy.
The evaluation was aimed to be directed both for the estimation of eradication rate (histology + bacterial culture) and plasma serology.

All examinations have been performed 6 weeks, 6 months and 12 months after the termination of the therapy and the results compared with the standard Losec + Amoxicillin treatment.

### PATIENTS AND TREATMENT

(A) Sixty-three patients with a mean age at 47.4 years (range 24.2-60.7) who were examined for gastro-eosophageal reflux disease (GERD) and shown by histology bacterial culture or both to be infected with Helicobacter pylori (HP) were included and randomly divided into three groups. Group 1 received Omeprazol plus Amoxicillin (20 mg twice daily and Amoxicillin 500 mg three times daily).

Group 2 was given Biogastrin plus Amoxicillin (Biogastrin 500 mg and Amoxicillin 500 mg three times daily).

Group 3 was given Biogastrin only (500 mg three times daily and Tinidazol 50 mg twice daily).

The treatment as above has been conducted in all groups during two weeks. There was no significant difference in the age. body weight, duration and the severity of the GERD (gastroscopy, histological findings and symptomatology - in particular intensity and frequency of heartburn's).

The criteria for eradication of bacteria were absence of bacteria in followup biopsy specimens as verified histologically, by culture or by both means.

### EXAMINATIONS

Two mucosal samples were taken from the duodenal bulb, the Antrum, and the body for histology, and one from the antrum and corpus for bacterial culture. Haematoxylin-eosin ang Giemsa stains were used for histological examination.
Samples for Heliocobacter pylori cultures were taken in 0.5 ml of 20% glucose, ground in mortars and inoculated into selctive and non-selective apart plates within 4h.

Antibodies to Helicobacter pylori were measured separately for IgG, IgA and IgM by an enzyme immunoassay method. The antigens used were an acid glycerine extract and a bacterial sonicate obtained by ultracentrifugation from Helicobacter pylori strain NCTC 11637.
The absorbency readings were converted to reciprocals of the end-point titres. The end-point titres were the dilution's of the serum at the cut-off level defined by the optical densities of positive reference serum pools at constant dilution's. Separate reference pools were used for immunoglobulins A, G and M. They were placed on each microtitre plate and all samples from a patient were studied in threefold dilution's according to - Kosunen et al, Lancet. 1992:339:893-895.

The pre-treatment and follow-up samples were taken 6 (+/2) weeks and 6 (+/2) months after the start of therapy and included biopsy samples for histology and bacterial cultures and serum samples for circulating Helicobacter pylori antibodies.

All patients gave their informed consent after they have been informed about the study protocol. Only patients with dyspepsia and heartburn's for at least one to three months who had no treatment with colloidal bismuth substrate, antibiotics, H₂ receptor, antagonists or Omeprazol (protein transport blockers) have been included in the study. Patients with a recent history of inspected or proven gastrointestinal bleeding or a part history of gastric surgery were also excluded.
The treatment was given and good tolerated during the whole period of two weeks. Two patients have dropped out due to unrelated family resons.

### RESULTS

Before therapy all 63 patients harboured bacteria and had histologically and gastropically verified active chronic gastritis and eosophagitis. Nearly all patients had raised titres of circulating Helicobacter pylori antibodies. Raised titres of IgG antibodies were found in 98% of patients, IgA in 94% and IgM in 48% of the patients.

In group 1 (Omeprazol + Amoxicillin) eighty-two percent of the patients reported a major improvement with amelioration of heartburn's and dyspepsia.
In group 2 (Biogastrin + Amoxicillin) the corresponding number was eighty percent.
In group3 (Biogastrin + Tinidazol) eigthy-four percent (for Metronizadol/Tinidazol sensitive and 68% for Mitronizadol resistant patients).

Six weeks after termination of treatment Helicobacter pylori bacteria were eradicated in 74% in patients belonging to group 1 and in 72% in group 2. In group 3 the eradication of Helicobacter pylori was found to reach 82% among patients infected with HP Metronizadol/Tinidazol sensitive strains and in 54% among patients with HP showing Metronizadol/Tinidazol insensitivity.

IgG antibodies had fallen slightly by 6 weeks in 70% of patients in group 1, and in 72% in group 2. In group 3 a significant fall was found in 78% of Metronizadol sensitive and in 48% among Metronizadol insensitive patients.

IgA titres decreased in 64% of patients in group 1, 72% in group 2 and 60% in group 3 (78% among Metronizadol) sensitive and 43% among Metronozadol resistant patients).

6 months after the termination of therapy IgG titres in bacteria negative patients were average 22% of the pre-treatment values and IgA titres 36%.
12 months samples from bacteria negative patients showed that antibody titres declined continuously. In most of the patients the fall in IgG and IgA titres was greater than 50%.

Eight patients among those who became symptomfreee in group 1 suffered severe heartburn's after the termination of the therapy and had tp start alternative therapy (Gaviscone and Sulfacrate) during the first 6 month period. The same occurred in 2 patients in group 2 and 4 patients in group 3. All the last patients belonged to Metronidazol resistant - HP positive patients at 6 months test.

IgM decreased in 67% of patients in group 1, 71% in group 2 and 64% in group 3 (but in 74% of Metronidazol sensitive patients).

In bacteria negative patients antibody titres continued to fall beyond the first 6 weeks, in bacteria positive titres remained high throughout the observation period.

Six months after the termination of the treatment only 64% of the patients in group 1 were still HP negative. The percent of HP negative patients for group 2 was still unchanged 72% and in group 3 decreased from 54% to 45% in Metronidazol) resistant patients remaining unchanged high in Metronidazol sensitive patients - 80%.

12 months after therapy the eradication rate was 56% for group 1, 72% for group 2 and 42 and 80% in group 3 for Metronizadol resistant and sensitive patients respectively.

### DISCUSSION

The results of the present study clearly show the high sensitivity of 50% fall in IgG titre as an indicator of bacterial eradication already at 6 weeks. The sensitivity of this test increased with time and the decrease became more specific at 6 and 12 months respectively.

Titres 40% or less of the pre-treatment value were seen only in bacteria negative patients directly after the termination of the treatment.

The effectiveness of all three therapies was comparable in term of amelioration of main symptoms associated with GERD when tested after 6 weeks.

Comparable was also the rate of eradication of HP (except for higher eradication rate for Metronizaadol/Tinidazol^{R} sensitive patients and lower in Metronidazol resistant patients treated with Biogastrozol).

However, patients in group 1 (Omeprazol + Amoxicillin^{R}) showed signigicant higher rate of reinfection (or reoccurrence) of Helicobacter pylori.
The same was true for the detoriation of symptomatology and a need of reinstitution of the antireflux and antiacis treatment. To some extent similar situation was observed in the Metronidazol/Tinidazol^{R} resistant patients treated with a combination of Biogastrin and Tinidazol^{R}. 84% of the patients in group 1 and 26% of the patients in group 3 craved the institution of alternative therapy (Gaviscone + Sucralfat).

The results of the present study indicate therefore that the double therapy with Biogastrin in to prefer against an combination of Omeprazol + Amoxicillin^{R} because of higher eradication rate verified after 6 and 12 months after the termination of the 2 weeks treatment.

The results of the present study seem also indicate that double therapy with the combination of protein pump blockers and Ampicillin is not satisfactory on the long run and the triple therapy or double therapy with Biogastrin and Amoxicillin^{R} or Tinidazol^{R} giving much better long-term results with respect both to the eradication of Helicobacter pylori and amelioration of the main symptoms of GERD.

## Claims

1. Pharmaceutical preparation for the treatment of gastritis, reflux oesophagitis, duodenitis, dyspepsia and ulcer disease,
**characterized in,**
**that** it contains a mixture of a bismuth citrate and an alginate and any reaction products obtained, especially bismuth alginate.

2. Pharmaceutical preparation or system according to claim 1,
**characterized in,**
**that** it further contains one or more antibiotic substances.

3. Pharmaceutical preparation or system according to claim 2,
**characterized in,**
**that** said antibiotic substance(s) is effective against Campylobacter, especially Heliobacter pyrolis.

4. Pharmaceutical prepration or system according to claim 2,
**characterized in,**
**that** said antibiotic substance is Amoxicillin.

5. Pharmaceutical preparation or system according to claim 2, **characterized in,**
**that** said antibiotic substance is Metronizadol.

6. Pharmaceutical preparation according to any of the preceding claims.
**characterized in,**
**that** it further contains an agent, preferably sodium bicarbonate, that will cause neutralization of the gastric in the acid milieu and foaming of the bismuth alginate.

7. Pharmaceutical preparation or system according to any of the preceeding claims,
**characterized in,**
**that** it contains a slow-release agent.

8. Pharmaceutical preparation or system according to claims 6,
**characterized in,**
**that** the slow-release agent is calcium alignate.

9. Pharmaceutical preparation according to any of the preceding claims,
**characterized in,**
**that** the bismuth citrate is ammonium bismuth citrate or ammonium bismuth subcitrate.

10. Pharmaceutical preparation according to any of the preceding claims,
**characterized in,**
**that** the alginate is a sodium-, calcium and/or potassium alginate.

11. Method of manufacturing the pharmaceutical preparation according to claim 1,
**characterized in**,
mixing a bismuth citrate with an alginate to obtain bismuth alginate.

12. Method according to claim 11,
**characterized in,**
**that** calcium chloride is added to the reaction medium to create microencapsulation beads of calcium alginate.

13. Method according to any of claims 11 or 12,
**characterized in**,
adding to the reaction medium an agent such as sodium bicarbonate causing a neutralitation of the gastric acid milieu and a foaming effect.

14. Method according to any of claims 11-13,
**characterized in**,
combining the bismuth alginate with one or more antibiotic substances.

## Patentansprüche

1. Pharmazeutisches Präparat für die Behandlung von Gastritis, Rückfluß-Ösophagitis, Duodenitis, Dyspepsie und von Geschwürerkrankungen,
**dadurch gekennzeichnet, dass**
es ein Gemisch aus einem Wismutcitrat und einem Alginat und erzielte Reaktionsprodukte enthält, insbesondere Wismutalginat.

2. Pharmazeutisches Präparat oder System gemäß Anspruch 1,
**dadurch gekennzeichnet, dass**
es weiterhin eine oder mehrere antibiotische Substanzen enthält.

3. Pharmazeutisches Präparat oder System gemäß Anspruch 2,
**dadurch gekennzeichnet, dass**
die antibiotische Substanz/antibiotischen Substanzen wirksam ist/sind gegen Campylobacter, insbesondere Heliobacter pyrolis.

4. Pharmazeutisches Präparat oder System gemäß Anspruch 2,
**dadurch gekennzeichnet, dass**
die antibiotische Substanz Amoxicillin ist.

5. Pharmazeutisches Präparat oder System gemäß Anspruch 2,
**dadurch gekennzeichnet, dass**
die antibiotische Substanz Metronizadol ist.

6. Pharmazeutisches Präparat gemäß einem der vorstehenden Ansprüche,
**dadurch gekennzeichnet, dass**
es weiterhin einen Wirkstoff enthält, vorzugsweise doppeltkohlensaures Natrium, das eine Neutralisierung des Magensäuremilieus und ein Schäumen des Wismutalginats bewirkt.

7. Pharmazeutisches Präparat oder System gemäß einem der vorstehenden Ansprüche,
**dadurch gekennzeichnet, dass**
es einen Wirkstoff mit verlangsamter Freisetzung enthält.

8. Pharmazeutisches Präparat oder System gemäß Anspruch 6,
**dadurch gekennzeichnet, dass**
der Wirkstoff mit verlangsamter Freisetzung Kalziumalginat ist.

9. Pharmazeutisches Präparat gemäß einem der vorstehenden Ansprüche,
**dadurch gekennzeichnet, dass**
das Wismutcitrat Ammoniumwismutcitrat oder Ammoniumwismutsubcitrat ist.

10. Pharmazeutisches Präparat gemäß einem der vorstehenden Ansprüche,
**dadurch gekennzeichnet, dass**
das Alginat ein Natrium-, Kalzium und/oder Kaliumalginat ist.

11. Verfahren zur Herstellung des pharmazeutischen Präparats gemäß Anspruch 1,
**dadurch gekennzeichnet, dass**
Wismutcitrat mit einem Alginat gemischt wird, um Wismutalginat zu erhalten.

12. Verfahren gemäß Anspruch 11,
**dadurch gekennzeichnet, dass**
das Kalziumchlorid dem Reaktionsmittel hinzugefügt wird, um Mikroverkapselungsperlen aus Kalziumalginat zu erzeugen.

13. Verfahren gemäß Anspruch 11 oder Anspruch 12,
**dadurch gekennzeichnet, dass**
dem Reaktionsmittel ein Wirkstoff hinzugefügt wird, wie z. B. doppeltkohlensaures Natrium, das eine Neutralisierung des Magensäuremilieus und eine schäumende Wirkung hervorruft.

14. Verfahren gemäß einem der Ansprüche 11-13,
**dadurch gekennzeichnet, dass**
das Wismutalginat mit einer oder mit mehreren antibiotischen Substanzen kombiniert wird.

## Revendications

1. Préparation pharmaceutique pour le traitement de gastrite, de reflux oesophagien, de duodénite, de dispepsie et d'ulcère,
**caractérisée en ce que**
elle contient un mélange d'un citrate de bismuth et d'un alginate et de quelconques produits de réaction obtenus, en particulier de l'alginate de bismuth.

2. Préparation ou système pharmaceutique selon la revendication 1,
**caractérisée en ce que**
elle contient de plus une ou plusieurs substances antibiotiques.

3. Préparation ou système pharmaceutique selon la revendication 2,
**caractérisée en ce que**
ladite ou lesdites substances antibiotiques sont efficaces contre un Campylobacter, en particulier Heliobacter pyrolis.

4. Préparation ou système pharmaceutique selon la revendication 2,
**caractérisée en ce que**
ladite substance antibiotique est l'Amoxicilline.

5. Préparation ou système pharmaceutique selon la revendication 2,
**caractérisée en ce que**
ladite substance antibiotique est le Metronizadol.

6. Préparation pharmaceutique selon l'une quelconque des revendications précédentes,
**caractérisée en ce que**
elle contient de plus un agent, de préférence du bicarbonate de sodium, qui va provoquer la neutralisation du composé gastrique dans le milieu acide et le moussage de l'alginate de bismuth.

7. Préparation ou système pharmaceutique selon l'une quelconque des revendications précédentes,
**caractérisée en ce que**
elle contient un agent de libération lente.

8. Préparation ou système pharmaceutique selon la revendication 6,
**caractérisée en ce que**
l'agent de libération lente est de l'alginate de calcium.

9. Préparation pharmaceutique selon l'une quelconque des revendications précédentes,
**caractérisée en ce que**
le citrate de bismuth est un citrate de bismuth d'ammonium ou un sous-citrate de bismuth d'ammonium.

10. Préparation pharmaceutique selon l'une quelconque des revendications précédentes,
**caractérisée en ce que**
l'alginate est un alginate de sodium, de calcium et/ou de potassium.

11. Procédé de fabrication de la préparation pharmaceutique selon la revendication 1,
**caractérisé par**
le mélange d'un citrate de bismuth avec un alginate pour obtenir de l'alginate de bismuth.

12. Procédé selon la revendication 11,
**caractérisé en ce que**
du chlorure de calcium est ajouté au milieu de réaction pour créer des billes de micro-encapsulation d'alginate de calcium.

13. Procédé selon l'une quelconque des revendications 11 ou 12,
**caractérisé par**
l'ajout au milieu de réaction d'un agent tel que du bicarbonate de sodium provoquant une neutralisation du milieu d'acide gastrique et un effet moussant.

14. Procédé selon l'une quelconque des revendications 11 à 13,
**caractérisé par**
la combinaison de l'alginate de bismuth avec une ou plusieurs substances antibiotiques.
